# FASCICULE DE BREVET EUROPEEN

(11) **EP 1 549 565 B1**
(45) Date de publication et mention de la délivrance du brevet: **08.03.2006**
(21) Numéro de dépôt: 03792446.1
(22) Date de dépôt: 12.08.2003
(51) Int. Cl.: B65D 83/00

(54) **DISTRIBUTEUR DE PRODUIT FLUIDE OU PULVERULENT**
ABGABEVORRICHTUNG FÜR FLUID ODER PULVERFöRMIGES PRODUKT
FLUID OR POWDERY PRODUCT DISPENSER

(30) Priorité: 23.08.2002 FR 0210550
(43) Date de publication de la demande: 06.07.2005
(73) Titulaire: VALOIS S.A.S., 27110 Le Neubourg (FR)
(72) Inventeur: DUQUET, Frédéric, F-27800 Thibouville (FR); GARCIA, Firmin, F-27000 Evreux (FR); MILIAN, Alex, F-27160 Breteuil sur Iton (FR)
(74) Mandataire: CAPRI
(86) Numéro de dépôt international: PCT/FR2003/002518
(87) Numéro de publication internationale: WO 2004/018320

(56) Documents cités:
- FR-A- 2 781 770
- FR-A- 2 791 645
- GB-A- 626 631
- US-A- 4 990 016
- US-A- 5 950 871

## Description

La présente invention concerne un distributeur de produit fluide, liquide ou pulvérulent, c'est-à-dire de liquide plus ou moins visqueux ou de poudre, contenu dans un réservoir. Le distributeur comprend en outre un orifice de distribution par lequel le produit est distribué. A cet effet, le réservoir comprend une paroi d'actionnement et une paroi de réaction, adaptées à être rapprochées l'une de l'autre par déformation élastique pour diminuer le volume du réservoir et ainsi refouler du produit à travers l'orifice de distribution. Ce genre de distributeur est fréquemment utilisé dans de nombreux domaines, et en particulier ceux de la cosmétique, de la parfumerie ou encore la pharmacie.

Il existe entre autre, des distributeurs de produit fluide de ce type utilisés dans le domaine de la parfumerie et de la cosmétique en tant qu'échantillon de produit, qui peuvent par exemple être en libre distribution pour que le consommateur ou la consommatrice puisse tester le produit avant de l'acheter. Les documents FR-A-2 778 639 et FR-A-2 791 645 décrivent de tels distributeurs à usage principal, mais pas exclusif, en tant qu'échantillon. Dans les distributeurs de ces documents, il est prévu deux films soudés ensemble, ou une coque soudée à un film, au niveau de leur périphérie pour définir un réservoir de produit. Ces documents prévoient également d'insérer une pièce de distribution entre les deux films ou la coque et le film. Cette pièce de distribution définit l'orifice de distribution et sert optionnellement de moyen de support pour une pièce de matière poreuse apte à s'imbiber de produit fluide. Pour l'actionnement de ces distributeurs, les films ou la coque définissent une paroi d'actionnement déformable sur laquelle on peut appuyer à l'aide d'un doigt ou de plusieurs doigts. Dans le cas où le distributeur comprend deux films soudés ensemble, il y a deux parois d'actionnement et on peut par exemple actionner les distributeurs en le maintenant entre le pouce et un ou plusieurs doigts de la même main. La paroi en contact avec le pouce définit ainsi une paroi d'actionnement, alors que la paroi en contact avec l'autre ou les autres doigts de la même main sert de paroi de réaction permettant d'exercer une poussée sur la paroi en contact avec le pouce. De manière similaire, lorsque le distributeur comprend une coque reliée à un film, le pouce vient en contact de la coque qui définit la paroi d'actionnement alors que les autres doigts de la même main viennent en contact du film operculaire qui sert ainsi de paroi de réaction pour permettre la poussée sur la paroi d'actionnement à l'aide du pouce. Ainsi, dans ces deux distributeurs de l'art antérieur, la paroi d'actionnement et la paroi de réaction sont formées par deux pièces séparées, alors que l'orifice de distribution est formé par une troisième pièce.

Dans un domaine technique quelque peu éloigné, le document GB-A-2 626 631 décrit une boîte d'insecticide permettant la pulvérisation de la poudre qu'elle contient. Pour ce faire, la boîte se présente sous la forme d'un soufflet définissant deux parois d'actionnement que l'on peut rapprocher par pivotement autour d'une partie de liaison qui définit l'orifice de pulvérisation. Toutefois, pour conférer à cette boîte une caractéristique d'élasticité ou de rappel, un ressort métallique est disposé à l'intérieur de la boîte de manière à solliciter les deux parois d'actionnement en éloignement l'une de l'autre. Ainsi, en position de repos, les deux parois d'actionnement sont éloignées au maximum, et on peut les rapprocher en comprimant le ressort situé à l'intérieur de la boîte. Dans ce document, les deux parois d'actionnement sont formées par deux morceaux de carton reliés à une de leurs extrémités à un morceau de bois définissant un trou dans lequel est inséré une buse métallique. Le ressort en forme de « V » est inséré à l'intérieur des deux morceaux en carton et le tout est enveloppé dans une ou plusieurs couches de matériaux flexibles, par exemple une feuille. Ainsi, cette feuille flexible recouvre les deux morceaux de carton et définit de ce fait les deux parois d'actionnement. En tout, le distributeur de l'art antérieur décrit dans ce document comprend six éléments constitutifs distincts.

La présente invention a pour but de remédier aux inconvénients présentés par l'art antérieur précité en définissant un nouveau distributeur de produit fluide ou pulvérulent fonctionnant également sur le principe mécanique du soufflet, mais incorporant un nombre minimum de pièces constitutives de sorte que son montage est très simple.

Pour ce faire, la présente invention prévoit un distributeur de produit fluide, liquide ou pulvérulent, comprenant un réservoir destiné à contenir du produit, et un orifice de distribution par lequel le produit est distribué, ledit réservoir comprenant une paroi d'actionnement et une paroi de réaction, adaptées à être rapprochées par déformation élastique pour diminuer le volume du réservoir et ainsi refouler du produit à travers l'orifice de distribution, le distributeur comprenant en outre un corps monobloc et au moins un film operculaire souple, la paroi d'actionnement et la paroi de réaction étant formées par le corps monobloc, caractérisé en ce que les parois d'actionnement et de réaction soient reliées ensemble en faisant un angle qui diminue lorsqu'on les rapproche l'une de l'autre. Avantageusement, l'orifice de distribution est formé par le corps monobloc. Ainsi, le corps monobloc forme déjà à lui seul les parois d'actionnement et de réaction ainsi que l'orifice de distribution. De préférence, la déformation élastique est assurée par le corps monobloc qui présente une résilience élastique apte à le ramener vers une position de repos, dans laquelle la paroi d'actionnement est la plus éloignée de la paroi de réaction. Ainsi, le corps monobloc remplit à lui seul la quasi-totalité des fonctions de distributeur, exceptée celle inhérente au film operculaire souple qui permet de compléter le réservoir tout en se déformant lors du rapprochement des parois d'actionnement et de réaction.

Selon une caractéristique avantageuse de l'invention, le corps définit une partie de tête (113 ; 213 ; 313 ; 413) qui relie la paroi d'actionnement à la paroi de réaction, la partie de tête restant sensiblement statique lors du rapprochement élastique des parois d'actionnement et de réaction, l'orifice de distribution étant formé par la partie de tête.

Dans ce cas, la paroi d'actionnement peut être articulée élastiquement sur la partie de tête. Avantageusement, la paroi d'actionnement est pivotante par rapport à la paroi de réaction. En variante, la paroi d'actionnement est élastiquement déformable. De préférence, la paroi de réaction est symétriquement identique à la paroi d'actionnement par rapport à la partie de tête.

Selon une autre caractéristique, les parois d'actionnement et de réaction convergent l'une vers l'autre au niveau de la partie de tête.

Selon un autre aspect, les parois d'actionnement et de réaction peuvent être sensiblement planes et rigides.

Selon une autre caractéristique intéressante de l'invention, qui comprend une pièce de matière poreuse apte à s'imprégner de produit, ladite pièce étant disposée de manière adjacente à l'orifice de distribution. Avantageusement, le corps définit une partie de tête formant l'orifice de distribution, ladite partie formant des moyens de retenue pour maintenir la pièce de matière poreuse adjacente à l'orifice de distribution. La pièce de matière poreuse est particulièrement utile dans le cas où le produit est un liquide, mais on peut très bien imaginer des formes de réalisation dans lesquelles on utilise également une pièce de matière poreuse apte à s'imprégner d'un produit pulvérulent avant sa distribution à travers l'orifice de distribution.

Selon un autre aspect, la pièce de tête forme un embout allongé à l'extrémité libre duquel est formé l'orifice de distribution. Cet embout allongé est particulièrement utile lorsque le distributeur sert de pulvérisateur, par exemple nasal, dans lequel l'embout est destiné à être inséré à l'intérieur d'un orifice du corps, par exemple les narines.

Selon une forme pratique de réalisation, les parois d'actionnement et de réaction sont reliées par des pans latéraux déformables.

Selon un autre aspect, le corps comprend au moins une zone périphérique de fixation plane sur laquelle un film operculaire plan est fixé. Ceci est notamment le cas lorsque les parois d'actionnement et de réaction sont pourvues de pans latéraux de liaison déformable. Dans ce cas, un seul film operculaire plan est suffisant.

Le corps peut par exemple être réalisé par injection-moulage, mais il peut également être réalisé à partir d'une plaque plane découpée puis coudée, l'orifice de distribution étant situé au niveau du coude. En variante, le corps peut également être réalisé à partir d'un profilé extrudé.

Selon un autre mode de réalisation, les parois d'actionnement et de réaction sont reliées par une bride de liaison élastiquement déformable formée par le corps monobloc. Cette liaison a pour fonction de conférer à elle seule ou d'améliorer la caractéristique de déformation élastique du corps permettant de ramener les parois d'actionnement et de réaction dans leur position de repos.

Un tel distributeur peut être constitué uniquement du corps monobloc, d'un ou de deux films operculaires et optionnellement d'une pièce de matière poreuse apte à s'imprégner de produit. On peut donc envisager un distributeur uniquement constitué du corps monobloc et d'un seul film operculaire, la pièce de matière poreuse pouvant être omise dans certains cas d'application.

L'invention sera maintenant plus amplement décrite en référence au dessin joint donnant, à titre d'exemple non limitatif, quatre modes de réalisation de l'invention.

Sur les figures :
la figure 1 est une vue en perspective d'un distributeur selon un premier mode de réalisation de l'invention,
la figure 2 est une vue en perspective du distributeur de la figure 1 avec le film operculaire retiré,
la figure 3 est une vue en coupe transversale verticale à travers le distributeur de la figure 1,
la figure 4 est une vue de dessus du distributeur de la figure 1,

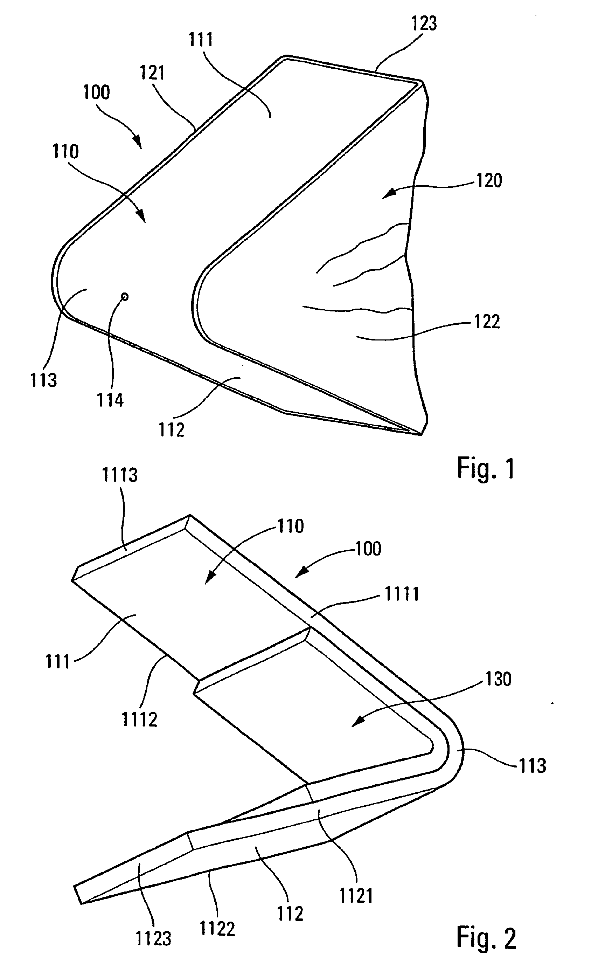

la figure 2 est une vue en perspective d'un distributeur selon un deuxième mode de réalisation de l'invention,

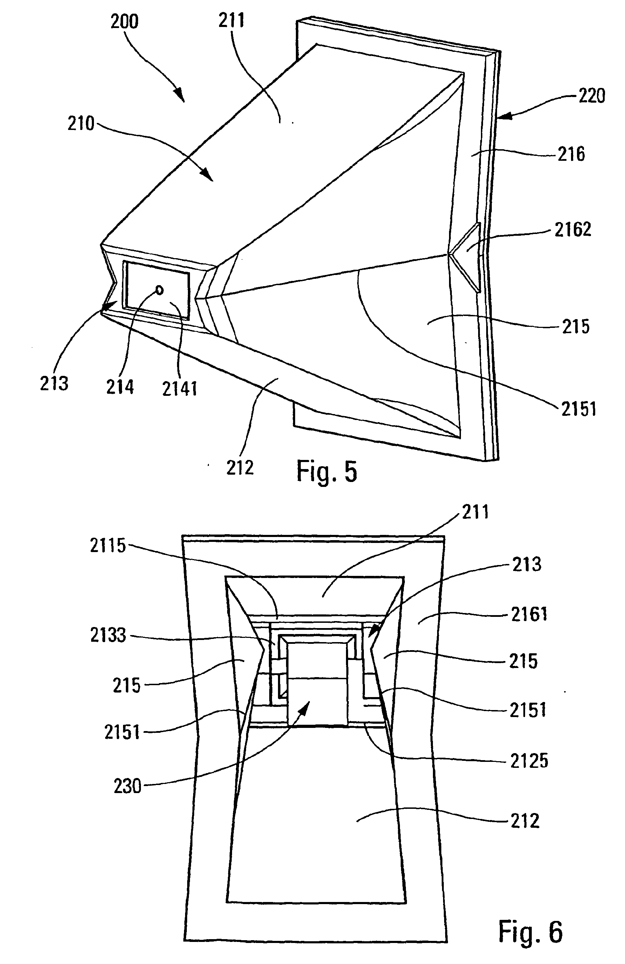

la figure 6 est une vue à l'intérieur du distributeur de la figure 5 lorsque le film operculaire est retiré,

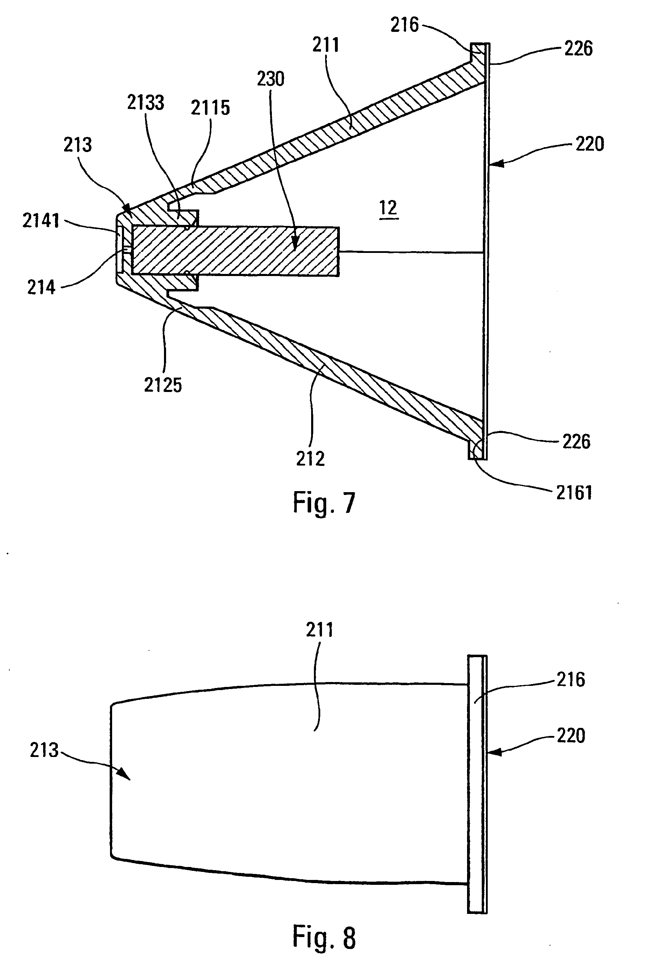

la figure 7 est une vue en coupe transversale verticale à travers le distributeur de la figure 5,
la figure 8 est une vue de dessus du distributeur de la figure 5,

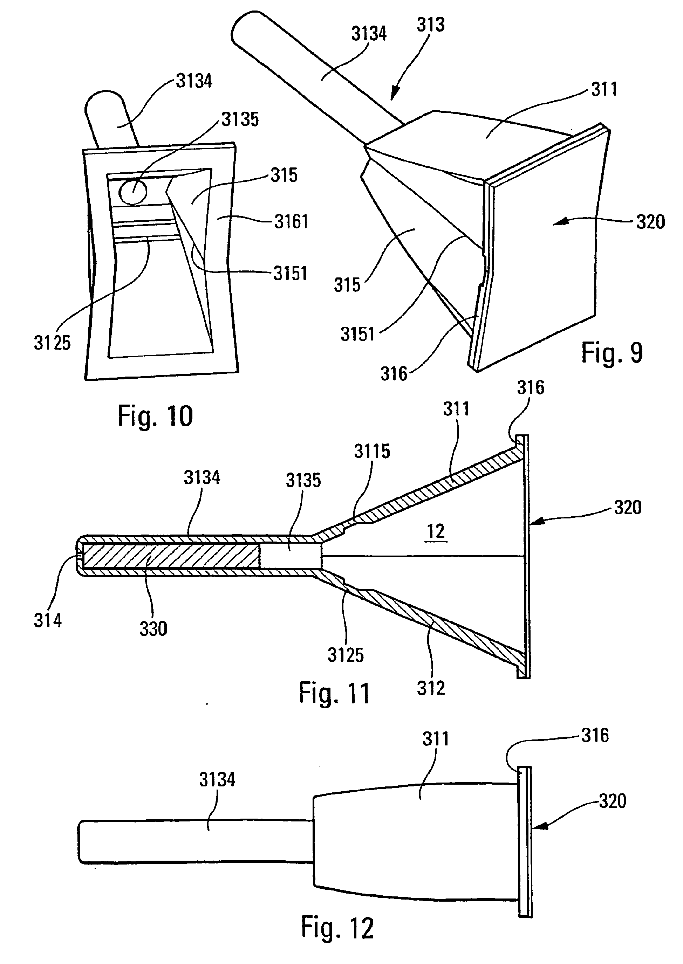

la figure 9 est une vue en perspective d'un distributeur selon un troisième mode de réalisation de l'invention,
la figure 10 est une vue à l'intérieur du distributeur de la figure 9 lorsque le film operculaire est retiré,
la figure 11 est une vue en coupe transversale verticale à travers le distributeur de la figure 9,
la figure 12 est une vue de dessus du distributeur de la figure 9,

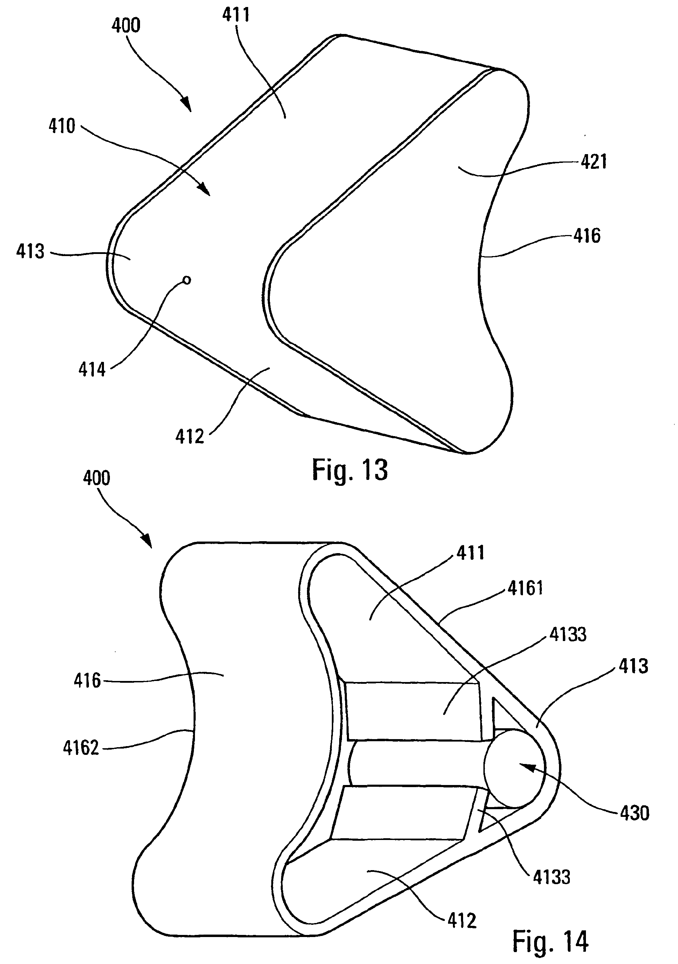

la figure 13 est une vue en perspective d'un distributeur selon un quatrième mode de réalisation de l'invention,
la figure 14 est une vue du distributeur de la figure 13 lorsque au moins un film operculaire a été retiré,

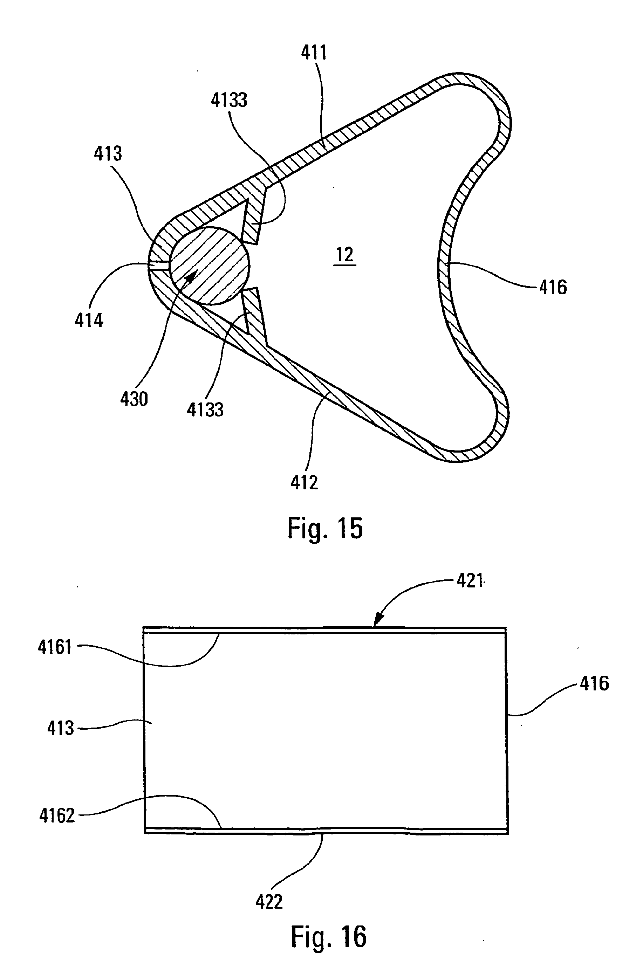

la figure 15 est une vue en coupe transversale verticale à travers le distributeur de la figure 13,
la figure 16 est une vue de dessus du distributeur de la figure 13.

Dans les quatre modes de réalisation qui vont être décrits ci-après, les distributeurs sont plutôt appropriés à distribuer un produit fluide liquide, mais ils peuvent également distribuer un produit fluide pulvérulent. Les quatre modes de réalisation présentent une forme générale de soufflet comprenant une paroi d'actionnement sur laquelle on peut appuyer par exemple à l'aide du pouce et une paroi de réaction qui peut être en contact d'un ou de plusieurs doigts de la même main, de sorte que l'on peut rapprocher la paroi d'actionnement de la paroi de réaction en appuyant sur la paroi d'actionnement à l'aide du pouce. Toutefois, un distributeur selon l'invention peut également être actionné par d'autres moyens qu'une main, par exemple, un dispositif mécanique. De même, dans les quatre modes de réalisation, les distributeurs définissent un réservoir contenant à la fois un produit fluide liquide ou pulvérulent ainsi qu'un gaz, en général de l'air. Bien entendu, d'autres gaz, par exemple des gaz inertes, peuvent être employés dans certains cas d'application, particulièrement en pharmacie. Du fait que le réservoir contient à la fois un produit fluide et du gaz, la distribution réalisée par un distributeur selon l'invention se présente sous la forme d'un jet pulvérisé biphasique dans lequel de fines gouttelettes de produit fluide sont transportées par un flux d'air éjecté sous pression. Toutefois, le réservoir peut également contenir uniquement du produit fluide, de sorte que la distribution se présente alors sous la forme d'un filet continu ou d'un jet pulvérisé ou encore sous la forme d'une goutte que l'on récupère par exemple à l'aide d'un doigt.

En se référant maintenant plus particulièrement aux figures 1 à 4 qui illustrent le premier mode de réalisation de l'invention, on voit que ce distributeur 100 comprend trois pièces constitutives, à savoir un corps monobloc 110, un film operculaire 120, et une pièce de matière poreuse 130. Dans certains cas d'application, on peut se passer de la pièce de matière poreuse 130, de sorte que le distributeur n'est alors constitué que de deux éléments constitutifs, à savoir le corps monobloc 110 et le film operculaire 120.

Le corps monobloc 110 présente une forme générale de bilame comprenant une première lame 111 reliée à une seconde lame 112 par une partie de liaison 113. La forme des lames 111, 112 peut être quelconque, c'est-à-dire polygonale ou arrondie. Quant à la partie de liaison 113, elle est de préférence coudée de manière arrondie sans former de pli net. On peut cependant imaginer une partie de liaison 113 présentant une paroi sensiblement plane délimitée par deux arêtes latérales à partir desquelles s'étendent les deux lames 111 et 112. Dans la forme de réalisation utilisée pour illustrer la présente invention, les lames 111 et 112 présentent chacune une forme sensiblement rectangulaire allongée de sorte que chaque lame est connectée à la partie de liaison 113 par un petit côté du rectangle. L'autre petit côté du rectangle définit une extrémité libre de lame. Chaque lame comprend ainsi trois bords. Plus précisément, la lame 111 comprend deux bords latéraux 1111 et 1112 et un bord d'extrémité 1113. De manière symétrique, la lame 112 présente deux bords latéraux 1121 et 1122 ainsi qu'un bord d'extrémité 1123. Ces bords présentent de préférence une certaine largeur qui définit ici l'épaisseur des lames. On peut cependant imaginer les formes de réalisation dans lesquelles chaque lame comprend des rabats définissant les bords des lames. Dans l'exemple représenté sur les figures 1 à 4, la hauteur des bords correspond à l'épaisseur des lames. D'ailleurs, cette épaisseur est constante au niveau des deux lames mais également au niveau de la partie de liaison 113. En d'autres termes, le corps monobloc présente une épaisseur de paroi constante. Toutefois, on peut imaginer des variantes de réalisation dans lesquelles l'épaisseur des lames ainsi que de la partie de liaison varie : par exemple, la partie de liaison 113 peut présenter une épaisseur supérieure à celle des lames au niveau des bords d'extrémité 1113 et 1123. D'autre part, dans le mode de réalisation représenté sur les figures 1 à 4, le corps monobloc 110 est parfaitement symétrique par rapport à la partie de liaison 113, de sorte que les lames 111 et 112 sont symétriquement identiques. On peut très bien prévoir que les deux lames 111 et 112 ne soient pas identiques. Par exemple, on peut prévoir que la lame 112 présente une épaisseur de paroi ou une forme différente de la lame 111. On peut également imaginer que la lame 112 soit reliée à la partie de liaison 113 d'une manière différente de celle de la lame 111.

Le corps monobloc 110 présente une caractéristique de déformation élastique qui se traduit de telle sorte que la lame 111 peut être rapprochée de la lame 112 à partir d'une position de repos, qui est celle représentée sur les figures 1 à 3. Dans cette position de repos, la lame 111 fait un angle maximal par rapport à la lame 112. En d'autres termes, la lame 111 converge vers la lame 112 au niveau de la partie de liaison 113 qui les relie ensemble. La résilience élastique du corps monobloc 110 est conférée en partie ou en totalité par la partie de liaison 113, dont l'arrondi peut être accentué par déformation élastique. Les lames 111 et 112 sont de préférence planes et rigides de sorte qu'elles peuvent pivoter autour de la partie de liaison 113 sensiblement sans se déformer. On peut également imaginer des formes de réalisation dans lesquelles les lames 111 et 112 présentent également une caractéristique de déformation élastique de sorte qu'elle participe ou qu'elle confère la caractéristique de déformation élastique du corps monobloc 110. On peut dans ce dernier cas prévoir une partie de liaison 113 sensiblement rigide et indéformable. On peut ainsi comprendre que le corps monobloc 110 présente une certaine résilience élastique qui lui est intrinsèque ou inhérente, sans qu'il soit pour autant important de savoir quelle partie du corps monobloc confère cette caractéristique de déformation élastique. On peut en effet même imaginer que le corps monobloc forme une ou plusieurs pattes souples qui s'étendent entre les deux lames 111 et 112 et qui confèrent ou améliorent la déformabilité élastique du corps monobloc. Ainsi, de quelque manière que soit réalisée cette caractéristique de déformation élastique, il est important que les deux lames 111 et 112 puissent être rapprochées l'une de l'autre à partir de la position de repos en surmontant la résistance élastique inhérente du corps monobloc. En pratique, le corps monobloc 110 peut être saisi à l'aide d'une main de sorte que la lame 111 vienne en contact du pouce et la lame 112 en contact d'un ou de plusieurs doigts de la même main qui sont ainsi en opposition par rapport au pouce. On peut ainsi exercer une pression sur la lame 111 à l'aide du pouce, de sorte que la lame 111 fait fonction de paroi d'actionnement, alors que la paroi 112 qui reste en appui sur un ou plusieurs doigts de la même main fait fonction de paroi de réaction permettant au pouce de pouvoir exercer une pression sur la paroi d'actionnement. Ainsi, selon l'invention, la lame 111 forme une paroi d'actionnement alors que la lame 112 forme une paroi de réaction. Quant à la partie de liaison 113, elle forme une partie de tête définissant un orifice de distribution 114. On peut également remarquer que lors du rapprochement des deux parois l'une de l'autre, la partie de tête 113 reste sensiblement statique de sorte que l'orifice de distribution reste également statique. Dès que l'on relâche la pression sur la paroi d'actionnement 111, le corps monobloc 110 revient bien entendu à sa position de repos du fait de la caractéristique de déformation élastique qui confère une mémoire de forme au corps monobloc.

Le corps monobloc 110 est de préférence réalisé en matière plastique, mais il peut également être réalisé en d'autres matériaux comme par exemple du métal. On peut aussi imaginer que le corps monobloc soit réalisé à partir de plusieurs matériaux, comme par exemple une lame de métal enrobée d'un autre matériau comme du plastique. Comme préalablement mentionné, le corps monobloc représenté sur les figures 1 à 3 présente une épaisseur de paroi constante, de sorte qu'il peut être très simplement réalisé en découpant une bande dans une plaque. Cette bande est ensuite repliée sur elle-même pour ainsi former les deux lames 111 et 112 ainsi que la partie de liaison 113 qui forme la pliure. Il suffit ensuite de ménager un orifice au niveau de la partie de liaison. On peut également imaginer que le corps monobloc soit réalisé à partir d'une sorte de cornière extrudée ou profilée, ou même moulée, dans laquelle la cornière définit deux brides longitudinales qui sont orientées l'une par rapport à l'autre avec un angle aigu et reliées par une partie de liaison. Il suffit alors de couper des tranches dans cette cornière de manière à obtenir un corps monobloc tel que représenté sur les figures 1 à 3. La solution utilisant la cornière permet de faire varier l'épaisseur de paroi du corps monobloc à ces différents endroits. On peut aussi imaginer que le corps monobloc soit réalisé par un procédé classique d'injection-moulage.

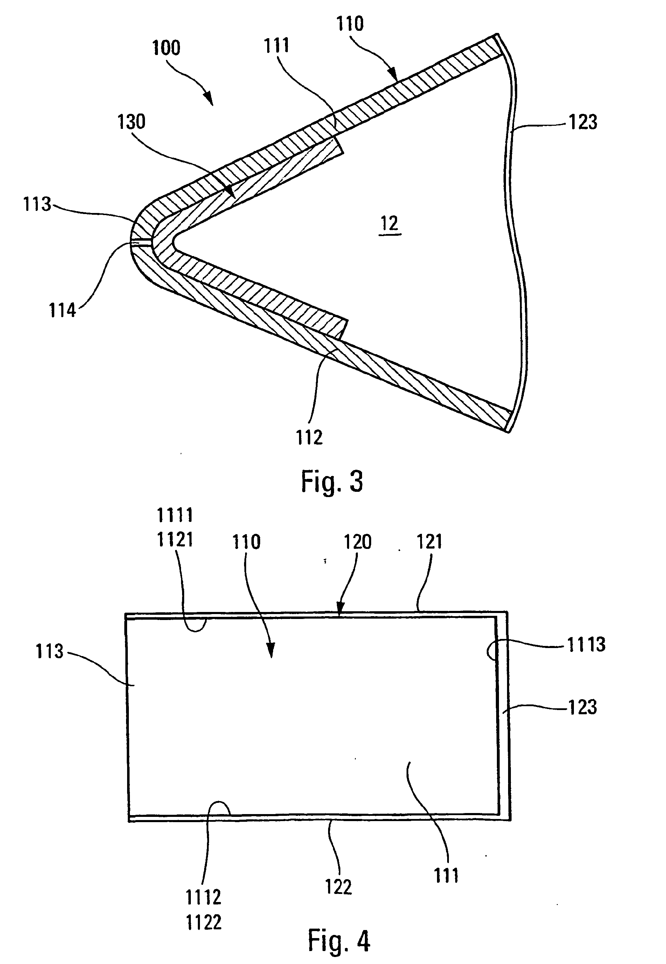

Optionnellement selon l'invention, le corps monobloc 110 peut être pourvu d'une pièce de matière poreuse 130 qui est disposée de manière adjacente à l'orifice de distribution 114, comme on peut le voir très clairement sur la figure 3. Cette pièce de matière poreuse 130 présente des caractéristiques d'imprégnabilité permettant à la matière poreuse de s'imprégner de produit fluide, qu'il soit liquide ou pulvérulent. Toutefois, l'utilisation d'une telle pièce de matière poreuse est plus particulièrement adaptée à la distribution de produit liquide. Dans l'exemple représenté sur les figures 2 et 3, la pièce de matière poreuse se présente sous la forme d'une bande ou languette qui est fixée entre les deux lames 110 et 111 par tout moyen technique approprié, comme par exemple le collage ou le soudage. La pièce de matière poreuse 130 s'étend par conséquent sur une partie des faces internes des lames 111 et 112 ainsi qu'au niveau de la face interne de la partie de liaison 113 où est formé l'orifice de distribution 114. Cette pièce de matière poreuse 130 peut être rapportée sur le corps monobloc 110 avant son pliage, lorsque le corps est réalisé à partir d'une plaque, ou même lors de la fabrication de la cornière susmentionnée. Il faut bien savoir que cette pièce de matière poreuse 130 n'est pas un élément constitutif essentiel de la présente invention, et qu'il est par conséquent possible de s'en passer dans certain cas d'application, particulièrement pour la distribution de produit pulvérulent.

Selon l'invention, le corps monobloc 110, éventuellement pourvu d'une pièce de matière poreuse 130, est pourvu d'un film operculaire 120 qui est fixé de manière étanche, par exemple par collage ou soudage, au corps monobloc 110 le long des bords 1111, 1112, 1113, 1121, 1122 et 1123 des lames 111 et 112. Il est important que la fixation du film operculaire 120 soit réalisée de manière étanche de manière à définir un volume interne étanche qui sert selon l'invention de réservoir de produit fluide 12. Ce réservoir peut être entièrement rempli de produit fluide, ou de préférence il peut être rempli d'une faible quantité de produit fluide, le restant du volume du réservoir étant rempli d'un gaz, par exemple de l'air. Le film operculaire 120 est souple et librement déformable sans trop d'élasticité ou de mémoire de forme, de sorte que les lames 111 et 112 peuvent être rapprochées l'une de l'autre en déformant ou en froissant le film operculaire 120. On se trouve alors en présence d'une sorte de soufflet classique, qui permet cependant de distribuer du produit fluide avec ou sans flux additionnel de gaz. Cependant, le soufflet n'est pas pourvu d'un clapet d'admission, l'air pénétrant à travers l'orifice de distribution à l'intérieur du réservoir 12 lors de la détente du corps monobloc 110 vers sa position de repos.

Pour obtenir une bonne fixation du film operculaire 120 sur les bords du corps monobloc 110, il est avantageux que ses bords présentent une certaine hauteur ou épaisseur, car ils définissent des zones de fixation ou d'application pour le film operculaire. La technique de fixation préférée est le soudage. On peut noter que les zones de fixation du film operculaire s'étendent sensiblement dans trois plans distincts, un premier plan formé par les bords 1111 et 1121, un second plan parallèle formé par les bords 1112 et 1122 et un troisième plan perpendiculaire formé par les bords 1113 et 1123, comme on peut le voir plus clairement sur la figure 4.

Ce distributeur selon ce premier mode de réalisation peut trouver une application privilégiée en tant qu'échantillon de produit fluide, par exemple à la disposition gratuite des consommateurs et consommatrices afin qu'ils puissent tester le produit avant un éventuel achat.

On se référera maintenant aux figures 5 à 8 qui représentent un deuxième mode de réalisation selon l'invention.

Ce deuxième mode de réalisation reprend certaines caractéristiques générales du premier mode de réalisation. Entre autre, le distributeur 200 dans ce second mode de réalisation comprend également un corps monobloc 210, éventuellement pourvu d'une pièce de matière poreuse 230, associée à un film operculaire 220. Le corps monobloc 210, qui est ici de préférence réalisé en matière plastique injectée-moulée, comprend deux lames 111 et 112 reliée par une partie de tête 213 dans laquelle est formé l'orifice de distribution 214. Cette partie de tête 213 sert également de liaison entre les deux lames 211 et 212. Les lames sont de préférence sensiblement planes et rigides et sont ici articulées au niveau de deux ponts 2115 et 2125 sur la tête 213 qui est ici sensiblement rigide. La tête forme une sorte de logement 2141 au fond duquel est formé l'orifice de distribution 214. Ce logement 2141 peut servir à recevoir un organe d'obturation amovible servant à obturer de matière étanche l'orifice de distribution 214 avant ou entre les utilisations. Intérieurement, la tête 213 forme des moyens de retenue 2133 servant à maintenir la pièce de matière poreuse 230 en place sur la tête 213 et entre les lames 211 et 212. Les ponts d'articulation 2115 et 2125 peuvent être élastiquement déformables, de sorte que les lames 211 et 212 ont alors tendance à revenir dans une position de repos, qui est celle représentée sur les figures 5 à 7. Toutefois, les ponts d'articulation peuvent également être parfaitement souples, c'est-à-dire sans mémoire de forme.

Selon l'invention, ce corps monobloc 210 est en outre pourvu de pans latéraux 215 qui relient les lames 211 et 212 entre elles. Plus précisément, un pan latéral 215 part de la tête 213 et fait la jonction entre les bords latéraux droits des lames 211 et 212. De manière symétrique, un autre pan latéral 215 part également de la tête 213 et fait la jonction entre les bords latéraux gauches des lames 211 et 212. De cette manière, le corps monobloc 210 forme une sorte de cornet conique à quatre faces formées par les deux lames 211 et 212 et les deux pans latéraux 215, la pointe étant formée par la partie de tête 213. Selon l'invention, chaque pan latéral 215 est déformable et de préférence élastiquement déformable, de sorte qu'il n'empêche pas le rapprochement des lames 211 et 212 par pivotement autour des ponts de matière 2115 et 2125. Au contraire, les pans latéraux 215 présentent de préférence une caractéristique de déformation élastique permettant de conférer au corps monobloc une fonction de rappel élastique, comme c'est le cas dans le premier mode de réalisation. Dans une forme de réalisation pratique, chaque pan latéral 215 peut être pourvu d'une ligne de pliage 2151 qui a tendance à se déplacer vers l'intérieur du corps monobloc lorsque les deux lames 111 et 112 sont rapprochées l'une de l'autre. Pour garantir le déplacement vers l'intérieur des lignes de pliage 2151, on peut remarquer que les pans latéraux 215 ne sont pas parfaitement plans, mais au contraire, légèrement incurvés vers l'intérieur pour prédéterminer le sens de déplacement des lignes de pliage 2151. Ainsi, en rapprochant la lame 211 de la lame 212, les deux lignes de pliage 2151 ont tendance à se rapprocher vers l'intérieur du corps monobloc 210. Ceci peut être aisément compris en regardant la figure 6. La caractéristique de rappel ou de déformation élastique du corps monobloc 210 peut être remplie uniquement par les pans latéraux 215, ou uniquement par les ponts de matière 2115 et 2125 ou une combinaison de ceux-ci. Dans ce second mode de réalisation, la partie de tête 213 ne participe pas ou pratiquement pas à la caractéristique de rappel ou de déformation élastique du corps monobloc 210.

A leurs extrémités opposées à la partie de tête 213, les lames 211 et 212 ainsi que les pans latéraux 215 forment une bordure 216 qui se présente ici sous la forme d'un cadre sensiblement rectangulaire, comme on peut le voir sur la figure 6. Cependant, les grands côtés du rectangle sont légèrement cassés vers l'intérieur du fait de l'orientation des lignes de pliure 2151. Toutefois, cette bordure 216 forme une surface ou une zone de fixation ou d'application 2161 qui est parfaitement plane, comme on peut le voir sur les figures 7 et 8. Cette surface de fixation ou d'application 2161, sert à la fixation d'un film operculaire 220 qui peut avantageusement être fixé au corps monobloc 210 en condition parfaitement plane. Le film operculaire 220 est fixé à la surface 261 au niveau de sa périphérie externe 226, comme on peut le voir sur la figure 7. Il est particulièrement avantageux que la surface ou zone de fixation 2161 du corps monobloc 210 soit parfaitement plane pour des raisons de technique de fixation ou de soudage, car l'élément applicateur peut ainsi être parfaitement plan.

Avec la fixation du film operculaire 220, il est ainsi défini un volume interne qui sert de réservoir 12, qui peut contenir uniquement du produit fluide liquide ou pulvérulent, ou également un gaz, comme de l'air. Comme dans le premier mode de réalisation, la pièce de matière poreuse 230 est en contact direct du produit fluide à l'intérieur du réservoir 12. Toutefois, on peut dans certains cas se passer de la pièce de matière poreuse 230. Ainsi, en appuyant sur la lame 211, qui sert de paroi d'actionnement, et en maintenant la lame 212 sensiblement fixe, qui sert alors de paroi de réaction, on peut rapprocher la lame 212 de la lame 211 par déformation souple des ponts 215 et 225, ce qui a pour effet de diminuer le volume interne du réservoir 12, du fait du déplacement des lignes de pliure 2151 des pans latéraux 215 et de la déformation souple du film operculaire 220. En effet, il est avantageux de choisir un film operculaire particulièrement souple, c'est-à-dire pratiquement sans mémoire de forme, de sorte qu'il peut être déformé, écrasé ou froissé sans générer de résistance mécanique. Bien entendu, tout comme dans le premier mode de réalisation, la diminution de volume de réservoir 12 a pour effet de mettre le produit fluide qu'il contient sous pression, de sorte qu'il n'a pas d'autre choix que d'être refoulé à travers l'orifice de distribution 214, et accessoirement en amont à travers la pièce de matière poreuse 230.

Le troisième mode de réalisation représenté sur les figures 9 à 12 est une variante de réalisation du mode précédent des figures 5 à 8. En effet, le corps monobloc 311 peut être identique à celui du second mode de réalisation en ce qui concerne les lames 311 ; 312 les ponts de matière 3115; 3125, les pans latéraux 315 avec leur ligne de pliure 3151, la bordure périphérique 316 avec sa surface d'application ou de fixation 3161. La différence avec le second mode de réalisation réside dans la partie de tête 313 qui forme ici un embout allongé 3134 à l'intérieur duquel s'étend un conduit 3135 contenant une pièce de matière poreuse 330. A l'extrémité de l'embout 3134, est formé un orifice de distribution 314. Cet embout de distribution 3134 permet de déporter l'orifice de distribution 314, de sorte que la distribution de produit peut être administrée dans des endroits difficiles d'accès, comme par exemple des orifices naturels du corps humain. On peut très bien utiliser un tel distributeur à embout pour des pulvérisations nasales dans lesquelles l'embout peut être inséré dans une narine du patient.

On se référera maintenant aux figures 13 à 16 qui représentent un quatrième mode de réalisation de l'invention. Ce distributeur 400 comprend 4 pièces constitutives, à savoir un corps monobloc 410, deux feuilles operculaires 421 et 422 et optionnellement une pièce de matière poreuse 430.

Le corps monobloc 410 comprend deux lames 411 et 412 reliées par une partie de liaison 413 formant un orifice de distribution 414. Les lames 411, 412 et la partie de liaison 413 avec l'orifice 414 peuvent se présenter extérieurement de manière sensiblement identique ou similaire à celle du premier mode de réalisation des figures 1 à 4. Les lames 411 et 412 sont de préférence sensiblement planes et rigides et la partie de liaison 413 peut se présenter sous une forme courbée arrondie. La largeur des lames est avantageusement identique à la largeur de la partie de liaison 413. Toutefois, comme on peut le voir plus clairement sur les figures 14 et 15, l'épaisseur de paroi des lames 411 et 412 n'est pas constante sur la longueur des lames : en effet, on peut voir que l'épaisseur diminue à mesure qu'on s'éloigne de la partie de liaison 413. De cette manière, les lames 411 et 412 présentent une certaine déformabilité élastique qui s'ajoute à celle de la partie de liaison 413. Ainsi, en appuyant sur les lames 411 et 412 de manière à les rapprocher, on déforme bien entendu la partie de tête 413 qui a tendance à accentuer son arrondi mais on déforme également l'extrémité des lames situées à l'opposé de la partie de tête 413.

Selon l'invention, les lames sont pourvues de brides 4133 qui définissent ensemble un logement à l'intérieur duquel est reçue une pièce de matière poreuse 430, qui se présente ici sous la forme d'un tronçon de cylindre. Ainsi, les brides 4133 font fonction de moyen de retenue permettant de maintenir la pièce de matière poreuse 430 de manière adjacente à l'orifice de distribution 414. Tout comme dans les modes de réalisation précédents, la pièce de matière poreuse 430 est apte à s'imprégner de produit fluide liquide ou pulvérulent avant son refoulement à travers l'orifice de distribution 14 lorsque l'on rapproche la lame 411 de la lame 412. Elle peut parfois être omise.

Dans cette forme de réalisation, les extrémités des lames opposées à la partie de tête ou de liaison 413 sont reliées entre elles par une bride de liaison élastiquement déformable 416, qui s'étend ici en forme d'arc de cercle. Cette bride de liaison élastique 416 participe à la caractéristique de déformation ou de rappel élastique du corps monobloc 410 : on peut même envisager que la bride de liaison 416 remplisse à elle seule cette fonction de rappel élastique. Avec cette bride de liaison 416, le corps monobloc 410 forme une boucle cylindrique refermée sur elle même. Ainsi, le corps monobloc 410 peut être réalisé à partir d'un profilé extrudé découpé en tranche ou tronçon à la largeur voulue. On peut également imaginer de former le corps monobloc 410 par injection-moulage.

Du fait de sa forme cylindrique fermée, le corps monobloc 410 forme deux bords ou tranches d'extrémité 4161 et 4162 qui s'étendent chacun dans un plan. De préférence, les deux plans ainsi formés sont parallèles l'un à l'autre. Ceci peut être remarqué très clairement sur la figure 16.

Selon l'invention, chaque bord d'extrémité est pourvu d'un film operculaire 421, 422 qui est fixé de manière étanche pour ainsi définir un volume interne qui sert de réservoir de produit fluide 12. Le produit fluide peut remplir la quasi-totalité du réservoir 12, ou en variant, le produit fluide peut partager le réservoir 12 avec un gaz, avantageusement de l'air, de manière à créer une pulvérisation biphasique à la sortie de l'orifice de distribution 414.

Il faut noter qu'il est particulièrement avantageux que les bords d'extrémité 4161 et 4162 s'étendent dans des plans, car ils servent de surface ou zone d'application ou de fixation pour les deux films operculaires 421 et 422. En effet, il est bien plus facile de fixer un film sur une surface plane que sur une surface profilée ou ondulée. Le distributeur dans ce mode de réalisation se présente alors, comme visible sur la figure 16, sous la forme d'une tranche épaisse constituée par le corps monobloc 410 bordé des deux côtés supérieurs et inférieurs par deux films operculaires 421 et 422 fixés de manière étanche.

Dans ce mode de réalisation, la caractéristique de déformation ou de rappel élastique peut être conférée individuellement ou cumulativement par la partie de tête ou de liaison 413, les lames 411, 412, la bride de liaison 416, ou encore les brides de retenue 4133 qui peuvent se déformer et ou venir en appui élastique sur la pièce de matière poreuse 430 qui peut également être déformée élastiquement par les extrémités des brides de retenues.

Tout comme dans les modes de réalisation précédents, la lame 411 définit une paroi d'actionnement alors que la lame 412 définit une paroi de réaction, ou inversement. On peut également dire que le distributeur définit deux parois d'actionnement définies par lames 411 et 412. Ceci est d'ailleurs valable pour les trois autres modes de réalisation précédents.

Dans ce quatrième mode de réalisation, comme dans les modes de réalisation précédents, les deux lames 411 et 412 sont symétriquement identiques. On peut toutefois imaginer une forme de réalisation dans laquelle les lames ont des formes différentes l'une de l'autre. On peut également imaginer que les lames aient des fonctions différentes : par exemple, la lame 411 ou la lame 412 peut être la seule paroi d'actionnement alors que l'autre lame constitue une véritable paroi de réaction dont le comportement dynamique par rapport à la tête est différent de l'autre lame.

Grâce à l'invention, un distributeur peut être réalisé avec des techniques très simples (injection-moulage, découpage-pliage, tronçonnage, collage ou soudage à plat) et avec un nombre de pièces très limité (4 au maximum et deux au minimum).

## Revendications

1. Distributeur de produit fluide, liquide ou pulvérulent (100 ; 200 ; 300 ; 400), comprenant un réservoir (12) destiné à contenir du produit, et un orifice de distribution (114 ; 214 ; 314 ; 414) par lequel le produit est distribué, ledit réservoir (12) comprenant une paroi d'actionnement (111 ; 211 ; 311 ; 411) et une paroi de réaction (112 ; 212 ; 312 ; 412), adaptées à être rapprochées par déformation élastique pour diminuer le volume du réservoir et ainsi refouler du produit à travers l'orifice de distribution, le distributeur comprenant en outre un corps monobloc (110 ; 210 ; 310 ; 410) et au moins un film operculaire souple (120 ; 220 ; 320 ; 421, 422), la paroi d'actionnement et la paroi de réaction étant formées par le corps monobloc, **caractérisé en ce que** les parois d'actionnement et de réaction soient reliées ensemble en faisant un angle qui diminue lorsqu'on les rapproche l'une de l'autre.

2. Distributeur selon la revendication 1, dans lequel l'orifice de distribution est formé par le corps monobloc.

3. Distributeur selon les revendications 1 ou 2, dans lequel le corps définit une partie de tête (113 ; 213 ; 313 ; 413) qui relie la paroi d'actionnement à la paroi de réaction, la partie de tête restant sensiblement statique lors du rapprochement élastique des parois d'actionnement et de réaction, l'orifice de distribution étant formé par la partie de tête.

4. Distributeur selon la revendication 3, dans lequel la paroi d'actionnement (111 ; 211 ; 311 ; 411) est articulée élastiquement sur la partie de tête.

5. Distributeur selon les revendications 3 ou 4, dans lequel la paroi d'actionnement (111 ; 211 ; 311 ; 411) est élastiquement déformable.

6. Distributeur selon les revendications 4 ou 5, dans lequel la paroi de réaction (112 ; 212 ; 312 ; 412) est symétriquement identique à la paroi d'actionnement par rapport à la partie de tête.

7. Distributeur selon l'une quelconque des revendications 3 à 6, dans lequel les parois d'actionnement et de réaction convergent l'une vers l'autre au niveau de la partie de tête.

8. Distributeur selon l'une quelconque des revendications précédentes, dans lequel les parois d'actionnement et de réaction sont sensiblement planes et rigides.

9. Distributeur selon l'une quelconque des revendications précédentes, comprenant une pièce de matière poreuse (130 ; 230 ; 330 ; 430) apte à s'imprégner de produit, ladite pièce étant disposée de manière adjacente à l'orifice de distribution (114 ; 214 ; 314 ; 414).

10. Distributeur selon la revendication 9, dans lequel le corps définit une partie de tête (113 ; 213 ; 313 ; 413) formant l'orifice de distribution (114 ; 214 ; 314 ; 414), ladite partie formant des moyens de retenue (2133 ; 3134 ; 4133) pour maintenir la pièce de matière poreuse (130 ; 230 ; 330 ; 430) adjacente à l'orifice de distribution.

11. Distributeur selon la revendication 3, dans lequel la pièce de tête forme un embout allongé (3134) à l'extrémité libre duquel est formé l'orifice de distribution (314).

12. Distributeur selon l'une quelconque des revendications précédentes, dans lequel les parois d'actionnement et de réaction sont reliées par des pans latéraux déformables (215 ; 315).

13. Distributeur selon l'une quelconque des revendications précédentes, dans lequel le corps(210 ; 310 ; 410) comprend au moins une zone périphérique de fixation plane (2161 ; 3161 ; 4161, 4162) sur laquelle un film operculaire plan (220 ; 320 ; 421, 422) est fixé.

14. Distributeur selon l'une quelconque des revendications précédentes, dans lequel le corps (210 ; 310) est réalisé par injection-moulage.

15. Distributeur selon l'une quelconque des revendications 1 à 13, dans lequel le corps (110) est réalisé à partir d'une plaque plane découpée puis coudée, l'orifice de distribution (114) étant situé au niveau du coude.

16. Distributeur selon l'une quelconque des revendications 1 à 13, dans lequel le corps (110 ; 410) est réalisé à partir d'un profilé extrudé.

17. Distributeur selon l'une quelconque des revendications précédentes, dans lequel les parois d'actionnement (411) et de réaction (412) sont reliées par une bride de liaison élastiquement déformable (416), formée par le corps monobloc (410).

18. Distributeur selon l'une quelconque des revendications précédentes, constitué uniquement du corps monobloc, d'un ou deux film(s) operculaire(s) et optionnellement d'une pièce de matière poreuse apte à s'imprégner de produit et disposée à proximité directe de l'orifice de distribution.

19. Distributeur selon l'une quelconque des revendications précédentes, dans lequel la déformation élastique est assurée par le corps monobloc qui présente une résilience élastique apte à le ramener vers une position de repos, dans laquelle la paroi d'actionnement est la plus éloignée de la paroi de réaction.

20. Distributeur selon l'une quelconque des revendications précédentes, dans lequel la paroi d'actionnement est pivotante par rapport à la paroi de réaction.

21. Distributeur selon la revendication 3, dans laquelle la partie de tête est élastiquement déformable.

22. Distributeur selon l'une quelconque des revendications précédentes, dans lequel le corps comprend deux lames (111, 112) définissant respectivement les parois d'actionnement et de réaction, les deux lames étant reliées l'une à l'autre au niveau d'une partie de liaison élastiquement déformable (113) de sorte que les deux lames pivotent l'une par rapport à l'autre par déformation élastique de la partie de liaison, les deux lames étant reliées l'une à l'autre par le film operculaire.

23. Distributeur selon l'une quelconque des revendications précédentes, dans lequel le corps (410) est constitué par un tronçon de cylindre déformable présentant deux tranches (4161, 4162) sur lesquelles sont fixées respectivement deux films operculaires (421, 422).

## Patentansprüche

1. Abgabevorrichtung für ein fluidförmiges, flüssiges oder pulverförmiges Produkt (100; 200; 300; 400), die einen Behälter (12), der dazu bestimmt ist, das Produkt zu enthalten, und eine Abgabeöffnung (114; 214; 314; 414) umfasst, durch welche das Produkt abgegeben wird, wobei der Behälter (12) eine Betätigungswand (111; 211; 311; 411) und eine Reaktionswand (112; 212; 312; 412) umfasst, die geeignet sind, durch elastische Verformung aufeinander zu bewegt zu werden, um das Volumen des Behälters zu vermindern und somit einen Teil des Produktes durch die Abgabeöffnung hindurch zu verdrängen, wobei die Abgabevorrichtung weiterhin einen einstückigen Körper (110; 210; 310; 410) und wenigstens einen nachgiebigen Abdeckfilm (120; 220; 320; 421, 422) umfasst, wobei die Betätigungswand und die Reaktionswand von dem einstückigen Körper gebildet werden, **dadurch gekennzeichnet, dass** die Betätigungswand und die Reaktionswand miteinander unter einem Winkel verbunden sind, der sich vermindert, wenn man sie aneinander annähert.

2. Abgabevorrichtung nach Anspruch 1, bei der die Abgabeöffnung im einstückigen Körper ausgebildet ist.

3. Abgabevorrichtung nach Anspruch 1 oder 2, bei der der Körper einen Kopfteil (113; 213, 313; 413) aufweist, der die Betätigungswand und die Reaktionswand miteinander verbindet, wobei der Kopfteil bei der elastischen Annäherung von Betätigungswand und Reaktionswand im wesentlichen statisch bleibt und die Abgabeöffnung in dem Kopfteil ausgebildet ist.

4. Abgabevorrichtung nach Anspruch 3, bei der die Betätigungswand (111; 211; 311; 411) elastisch am Kopfteil angelenkt ist.

5. Abgabevorrichtung nach Anspruch 3 oder 4, bei der die Betätigungswand (111; 211; 311; 411) elastisch verformbar ist.

6. Abgabevorrichtung nach Anspruch 4 oder 5, bei der die Reaktionswand (112; 212; 312; 412) mit der Betätigungswand bezüglich des Kopfteils symmetrisch identisch ist.

7. Abgabevorrichtung nach einem der Ansprüche 3 bis 6, bei der die Betätigungswand und die Reaktionswand im Bereich des Kopfteils zueinander hin konvergieren.

8. Abgabevorrichtung nach einem der vorhergehenden Ansprüche, bei der die Betätigungswand und die Reaktionswand im Wesentlichen flach und steif sind.

9. Abgabevorrichtung nach einem der vorhergehenden Ansprüche, die ein Element (130; 230; 330; 430) aus porösem Material umfasst, das sich mit dem Produkt voll saugen kann, wobei das Element benachbart zur Abgabeöffnung (114; 214; 314; 414) angeordnet ist.

10. Abgabevorrichtung nach Anspruch 9, bei der der Körper einen Kopfteil (113; 213; 313; 413) aufweist, in dem die Abgabeöffnung (114; 214; 314; 414) ausgebildet ist, wobei dieser Teil Halteeinrichtungen (2133; 3134; 4133) bildet, um das Element (130; 230; 330; 430) in der Nähe der Abgabeöffnung festzuhalten.

11. Abgabevorrichtung nach Anspruch 3, bei der das Kopfteil einen langgestreckten Ansatz (3134) aufweist, an dessen freiem Ende die Abgabeöffnung (314) ausgebildet ist.

12. Abgabevorrichtung nach einem der vorhergehenden Ansprüche, bei der die Betätigungswand und die Reaktionswand durch seitliche, verformbare Flächen (215; 315) verbunden sind.

13. Abgabevorrichtung nach einem der vorhergehenden Ansprüche, bei der der Körper (210; 310; 410) wenigstens eine ebene Befestigungs-Umfangszone (2161; 3161; 4161, 4162) umfasst, auf der ein ebener Abdeckfilm (220; 320; 421, 422) befestigt ist.

14. Abgabevorrichtung nach einem der vorhergehenden Ansprüche, bei der der Körper (210; 310) durch Spritzgießen hergestellt ist.

15. Abgabevorrichtung nach einem der Ansprüche 1 bis 13, bei der der Körper (110) ausgehend von einer zugeschnittenen, flachen und dann gebogenen Platte hergestellt ist, wobei die Abgabeöffnung (114) sich im Bereich der Biegung befindet.

16. Abgabevorrichtung nach einem der Ansprüche 1 bis 13, bei der der Körper (110; 410) aus einem extrudierten Profil hergestellt ist.

17. Abgabevorrichtung nach einem der vorhergehenden Ansprüche, bei der die Betätigungswand (411) und die Reaktionswand (412) durch einen elastisch verformbaren Verbindungssteg (416) verbunden sind, der vom einstückigen Körper (410) gebildet wird.

18. Abgabevorrichtung nach einem der vorhergehenden Ansprüche, die lediglich von dem einstückigen Körper und einem oder zwei Abdeckfilm(en) und optional einem Element aus porösem Material gebildet wird, das sich mit dem Produkt voll saugen kann und in unmittelbarer Nähe der Abgabeöffnung angeordnet ist:

19. Abgabevorrichtung nach einem der vorhergehenden Ansprüche, bei der die elastische Verformung durch den einstückigen Körper sichergestellt ist, der eine elastische Nachgiebigkeit besitzt, die ihn in eine Ruhelage zurückbringen kann, in welcher die Betätigungswand am weitesten von der Reaktionswand entfernt ist.

20. Abgabevorrichtung nach einem der vorhergehenden Ansprüche, bei der die Betätigungswand bezüglich der Reaktionswand verschwenkbar ist.

21. Abgabevorrichtung nach Anspruch 3, bei der der Kopfteil elastisch verformbar ist.

22. Abgabevorrichtung nach einem der vorhergehenden Ansprüche, bei der der Körper zwei Platten (111, 112) umfasst, die die Betätigungswand bzw. die Reaktionswand bilden, wobei diese beiden Platten miteinander im Bereich eines elastisch verformbaren Verbindungsteils (113) derart verbunden sind, dass die beiden Platten sich gegeneinander durch elastische Verformung des Verbindungsteils verschwenken, wobei die beiden Platten durch den Abdeckfilm miteinander verbunden sind.

23. Abgabevorrichtung nach einem der vorhergehenden Ansprüche, bei der der Körper (410) von einem stumpfkegeligen, verformbaren Zylinder gebildet wird, der zwei Abschnitte (4161, 4162) aufweist, an denen jeweils zwei Abdeckfilme (421, 422) befestigt sind.

## Claims

1. A fluid dispenser (100; 200; 300; 400) for dispensing a fluid in liquid or in powder form, said dispenser including a reservoir (12) serving to contain fluid, and a dispensing orifice (114; 214; 314; 414) via which the fluid is dispensed, said reservoir (12) having an actuating wall (111; 211; 311; 411) and a backing wall (112; 212; 312; 412), said walls be adapted to be brought towards each other by elastic deformation to reduce the volume of the reservoir, and thus to deliver fluid through the dispensing orifice, said dispenser further comprising a one-piece body (110; 210; 310; 410) and of at least one flexible sealing film (120; 220; 320; 421; 422), the actuating wall and the backing wall being formed by the one-piece body, **characterized in that** the actuating wall and the backing wall are angularly positioned relative to each by forming an angle that decreases when they are brought towards each other.

2. A dispenser according to claim 1, in which the dispensing orifice is formed by the one-piece body.

3. A dispenser according to claim 1 or 2, in which the body defines a head portion (113; 213; 313; 413) which connects the actuating wall to the backing wall, said head portion remaining substantially static while the actuating wall and the backing wall are being brought elastically towards each other, the dispensing orifice being formed by the head portion.

4. A dispenser according to claim 3, in which the actuating wall (111; 211; 311; 411) is hinged elastically to the head portion.

5. A dispenser according to claim 3 or claim 4, in which the actuating wall (111; 211; 311; 411) is elastically deformable.

6. A dispenser according to claim 4 or claim 5, in which the backing wall (112; 212; 312; 412) is symmetrically identical to the actuating wall about the head portion.

7. A dispenser according to any one of claims 3 to 6, in which the actuating wall and the backing wall converge mutually at the head portion.

8. A dispenser according to any preceding claim, in which the actuating wall and the backing wall are substantially plane and rigid.

9. A dispenser according to any preceding claim, including a piece of porous material (130; 230; 330; 430) suitable for being impregnated with fluid, said piece being disposed adjacent to the dispensing orifice (114; 214; 314; 414).

10. A dispenser according to claim 9, in which the body defines a head portion (113; 213; 313; 413) forming the dispensing orifice (114; 214; 314; 414), said portion forming retaining means (2133; 3134; 4133) for holding the piece of porous material (130; 230; 330; 430) adjacent to the dispensing orifice.

11. A dispenser according to claim 3, in which the head portion forms an elongate end-piece (3134) at the free end of which the dispensing orifice is formed (314).

12. A dispenser according to any preceding claim, in which the actuating wall and the backing wall are interconnected by deformable side panels (215; 315).

13. A dispenser according to any preceding claim, in which the body (210; 310; 410) is provided with at least one plane peripheral fixing zone (2161; 3161; 4161, 4162) to which a plane sealing film (220; 320; 421, 422) is fixed.

14. A dispenser according to any preceding claim, in which the body (210; 310) is made by injection molding.

15. A dispenser according to any one of claims 1 to 13, in which the body (110) is made from a plane sheet that is cut and then folded, the dispensing orifice (114) being situated at the fold.

16. A dispenser according to any one of claims 1 to 13, in which the body (110; 410) is made from an extruded shaped-section member.

17. A dispenser according to any preceding claim, in which the actuating wall (411) and the backing wall (412) are connected together via an elastically deformable coupling web (416) formed by the one-piece body (410).

18. A dispenser according to any preceding claim, made up only of the one-piece body, of one or more sealing films, and optionally of a piece of porous material suitable for being impregnated with fluid and disposed in the immediate vicinity of the dispensing orifice.

19. A dispenser according to any preceding claim, in which the elastic deformation is provided by the one-piece body which has elastic resilience suitable for returning it to a rest position, in which the actuating wall is as far away as it can be from the backing wall.

20. A dispenser according to any preceding claim, in which the actuating wall is mounted to pivot relative to the backing wall.

21. A dispenser according to claim 3, in which the head portion is elastically deformable.

22. A dispenser according to any preceding claim, in which the body comprises two blades (111, 112) respectively defining said actuating and backing walls, said two blades being connected together at a coupling portion elastically deformable (113) so that the blades pivot relative to each other by elastical deformation of the coupling portion, said two blades being connected together by the sealing film.

23. A dispenser according to any preceding claim, in which the body (410) is constituted by a segment of deformable cylinder having two edges (4161, 4162) on which two sealing films (421, 422) are respectively secured.
